(19) **Europäisches Patentamt / European Patent Office / Office européen des brevets**

(11) **EP 4 487 754 A1**

(12) **EUROPEAN PATENT APPLICATION**
published in accordance with Art. 153(4) EPC

(43) Date of publication:
**08.01.2025 Bulletin 2025/02**

(21) Application number: **22928742.0**

(22) Date of filing: **28.02.2022**

(51) International Patent Classification (IPC):
**A61B 1/045** (2006.01)

(52) Cooperative Patent Classification (CPC):
**A61B 1/045**

(86) International application number:
**PCT/JP2022/008253**

(87) International publication number:
**WO 2023/162216 (31.08.2023 Gazette 2023/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **NEC Corporation**
**108-8001 Tokyo (JP)**

(72) Inventors:
• **WATANABE, Kazuhiro**
**Tokyo 108-8001 (JP)**
• **SAIKOU, Masahiro**
**Tokyo 108-8001 (JP)**
• **IWADATE, Yuji**
**Tokyo 108-8001 (JP)**

(74) Representative: **Betten & Resch**
**Patent- und Rechtsanwälte PartGmbB**
**Maximiliansplatz 14**
**80333 München (DE)**

(54) **IMAGE PROCESSING DEVICE, IMAGE PROCESSING METHOD, AND STORAGE MEDIUM**

(57) The image processing device 1X includes an acquisition means 30X, a score calculation means 31X, and a classification means 32X. The acquisition means 30X acquires a captured image obtained by photographing an examination target by a photographing unit provided in an endoscope. The score calculation means 31X calculates, based on the captured images in time series obtained from a start time, a score regarding a likelihood of a presence of a region of interest in the captured images. Here, upon detecting a variation in the captured images, the score calculation means 31X sets a time after the variation as the start time. The classification means 32X classifies, based on the score, the captured images in time series if a predetermined condition is satisfied.

FIG. 13

```
              START
                │
  S31 ┌─────────▼──────────────────────────┐
      │ ACQUIRE CAPTURED IMAGE OF EXAMINATION TARGET │
      └─────────┬──────────────────────────┘
                │
  S32 ┌─────────▼──────────────────────────┐
      │ BASED ON CAPTURED IMAGES IN TIME SERIES OBTAINED │
      │ FROM START TIME, CALCULATE SCORE REGARDING REGION │
      │ OF INTEREST │
      └─────────┬──────────────────────────┘
                │
  S33      ◇ VARIATION IN CAPTURED IMAGES ◇──No──┐
           ◇     DETECTED ?              ◇       │
                │ Yes                            │
  S34 ┌─────────▼──────────────────────────┐    │
      │ SET TIME AFTER VARIATION AS START TIME │  │
      └─────────┬──────────────────────────┘    │
                │◄───────────────────────────────┘
  S35      ◇ PREDETERMINED CONDITION ◇──No──┐
           ◇     SATISFIED ?         ◇      │
                │ Yes                       │
  S36 ┌─────────▼──────────────────────────┐│
      │ CLASSIFY TIME SERIES CAPTURED IMAGES BASED ON SCORE │
      └─────────┬──────────────────────────┘
                │
               END
```

**Description**

TECHNICAL FIELD

The present disclosure relates to a technical field of an image processing device, an image processing method, and a storage medium for processing an image to be acquired in endoscopic examination.

BACKGROUND

An endoscopic examination system for displaying images taken in the lumen of an organ is known. For example, Patent Literature 1 discloses a learning method of a learning model that outputs information relating to a lesion part included in an endoscope image data when the endoscope image data generated by a photographing device is inputted thereto. Further, Patent Literature 2 discloses a classification method for classifying series data through an application method of the sequential probability ratio test (SPRT: Sequential Probability Ratio Test).

CITATION LIST

PATENT LITERATURE

Patent Literature 1: WO2020/003607
Patent Literature 1: WO2020/194497

SUMMARY

PROBLEM TO BE SOLVED

In the case of detecting a lesion part from images taken in the endoscopic examination, the detection of the lesion part is carried out by individually analyzing each image obtained by an endoscope. However, according to this technique, there is a possibility that existence of lesion parts which are difficult to be identified from a single image could not be detected properly. On the other hand, in detecting the existence of a lesion part, it is necessary to consider the influence caused in such a case where an image inappropriate for the identification is included in time series images.

In view of the above-described issue, it is therefore an example object of the present disclosure to provide an image processing device, an image processing method, and a storage medium capable of suitably detecting a region of interest such as a lesion part in an endoscopic examination.

MEANS FOR SOLVING THE PROBLEM

One mode of the image processing device is an image processing device including:

an acquisition means configured to acquire a captured image obtained by photographing an examination target by a photographing unit provided in an endoscope;
a score calculation means configured to calculate, based on the captured images in time series obtained from a start time, a score regarding a likelihood of a presence of a region of interest in the captured images; and
a classification means configured to classify, based on the score, the captured images in time series if a predetermined condition is satisfied,
wherein, upon detecting a variation in the captured images, the score calculation means is configured to set a time after the variation as the start time.

One mode of the image processing method is an image processing method executed by a computer, the image processing method including:

acquiring a captured image obtained by photographing an examination target by a photographing unit provided in an endoscope;
calculating, based on the captured images in time series obtained from a start time, a score regarding a likelihood of a presence of a region of interest in the captured images;
upon detecting a variation in the captured images, setting a time after the variation as the start time; and

classifying, based on the score, the captured images in time series if a predetermined condition is satisfied.

One mode of the storage medium is a storage medium storing a program executed by a computer, the program causing the computer to:

acquire a captured image obtained by photographing an examination target by a photographing unit provided in an endoscope;

calculate, based on the captured images in time series obtained from a start time, a score regarding a likelihood of a presence of a region of interest in the captured images;

upon detecting a variation in the captured images, set a time after the variation as the start time; and

classify, based on the score, the captured images in time series if a predetermined condition is satisfied.

EFFECT

An example advantage according to the present disclosure is to suitably detect a region of interest from captured images.

BRIEF DESCRIPTION OF THE DRAWINGS

[FIG. 1] It illustrates a schematic configuration of an endoscopic examination system.
[FIG. 2] It illustrates a hardware configuration of an image processing device.
[FIG. 3] It is a functional block diagram of the image processing device.
[FIG. 4] It is a graph showing the transition of the classification score calculated in the sequential calculation process.
[FIG. 5] It is a graph showing the transition of the classification score provided that a determination as to whether or not to reset the classification score in consideration of the threshold difference is made.
[FIG. 6] It is a graph showing the transition of the classification score calculated in the parallel calculation process.
[FIG. 7] It is a first display example of the display screen image displayed on the display device in the endoscopic examination.
[FIG. 8] It is a second display example of the display screen image displayed on the display device in the endoscopic examination.
[FIG. 9] It is a third display example of the display screen image displayed on the display device in the endoscopic examination.
[FIG. 10] It illustrates an example of a flowchart that the image processing device executes regarding the detection of the lesion part based on the sequential calculation process.
[FIG. 11] It illustrates an example of a flowchart that the image processing device executes regarding the detection of the lesion part based on the parallel calculation process.
[FIG. 12] It is a block diagram of an image processing device according to a second example embodiment.
[FIG. 13] It illustrates an example of a flowchart executed by the image processing device according to the second example embodiment.

EXAMPLE EMBODIMENTS

Hereinafter, example embodiments of an image processing device, an image processing method, and a storage medium will be described with reference to the drawings.

<First Example Embodiment>

(1) System Configuration

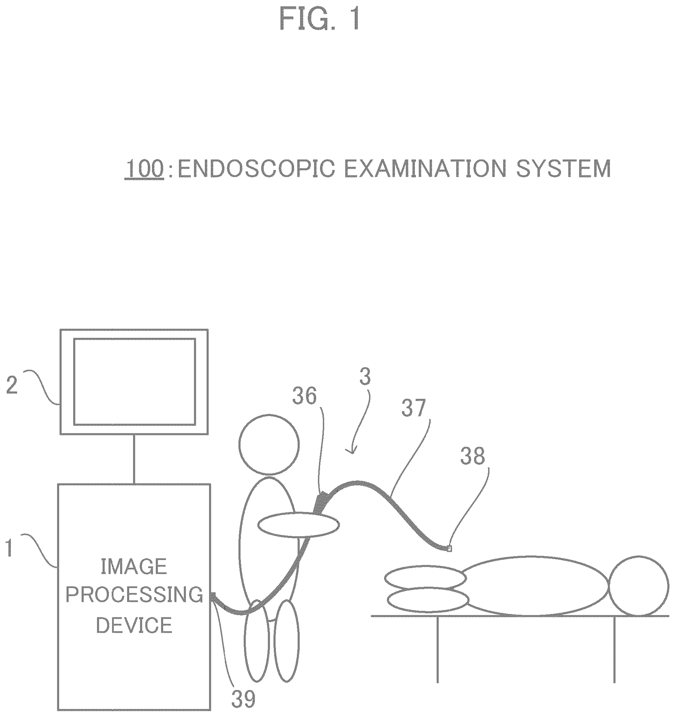

FIG. 1 shows a schematic configuration of an endoscopic examination system 100. As shown in FIG. 1, the endoscopic examination system 100 is a system for presenting a part (a lesion part) of examination target which is suspected of a lesion to an examiner such as a doctor who performs examination or treatment using an endoscope. The endoscopic examination system 100 mainly includes an image processing device 1, a display device 2, and an endoscope scope 3 connected to the image processing device 1.

The image processing device 1 acquires an image (also referred to as "captured image Ia") captured by the endoscope 3 in time series from the endoscope 3 and displays a screen image based on the captured image Ia on the display device 2. The captured image Ia is an image captured at predetermined time intervals in at least one of the insertion process of the endoscope 3 to the subject or the ejection process of the endoscope 3 from the subject. In the present example embodiment, the image processing device 1 analyzes the captured image Ia to detect a lesion part from the captured images Ia, and displays the information regarding the detection result on the display device 2.

The display device 2 is a display or the like for display information based on the display signal supplied from the image processing device 1.

The endoscope 3 mainly includes an operation unit 36 for examiner to perform a predetermined input, a shaft 37 which has flexibility and which is inserted into the organ to be photographed of the subject, a tip unit 38 having a built-in photographing unit such as an ultrasmall image pickup device, and a connecting unit 39 for connecting with the image processing device 1.

The configuration of the endoscopic examination system 100 shown in FIG.1 is an example, and various change may be applied thereto. For example, the image processing device 1 may be configured integrally with the display device 2. In another example, the image processing device 1 may be configured by a plurality of devices.

Hereafter, as a representative example, the description will be given of the process in the endoscopic examination of the large bowel. However, the examination target is not limited to the large bowel and it may be an esophagus or stomach. Examples of the target of the endoscopic examination in the present disclosure include a laryngendoscope, a bronchoscope, an upper digestive tube endoscope, a duodenum endoscope, a small bowel endoscope, a large bowel endoscope, a capsule endoscope, a thoracoscope, a laparoscope, a cystoscope, a cholangioscope, an arthroscope, a spinal endoscope, a blood vessel endoscope, and an epidural endoscope. In addition, the conditions of the lesion part to be detected in endoscopic examination are exemplified as (a) to (f) below.

(a) Head and neck: pharyngeal cancer, malignant lymphoma, papilloma
(b) Esophagus: esophageal cancer, esophagitis, esophageal hiatal hernia, Barrett's esophagus, esophageal varices, esophageal achalasia, esophageal submucosal tumor, esophageal benign tumor
(c) Stomach: gastric cancer, gastritis, gastric ulcer, gastric polyp, gastric tumor
(d) Duodenum: duodenal cancer, duodenal ulcer, duodenitis, duodenal tumor, duodenal lymphoma
(e) Small bowel: small bowel cancer, small bowel neoplastic disease, small bowel inflammatory disease, small bowel vascular disease
(f) Large bowel: colorectal cancer, colorectal neoplastic disease, colorectal inflammatory disease; colorectal polyps, colorectal polyposis, Crohn's disease, colitis, intestinal tuberculosis, hemorrhoids.

(2) Hardware Configuration

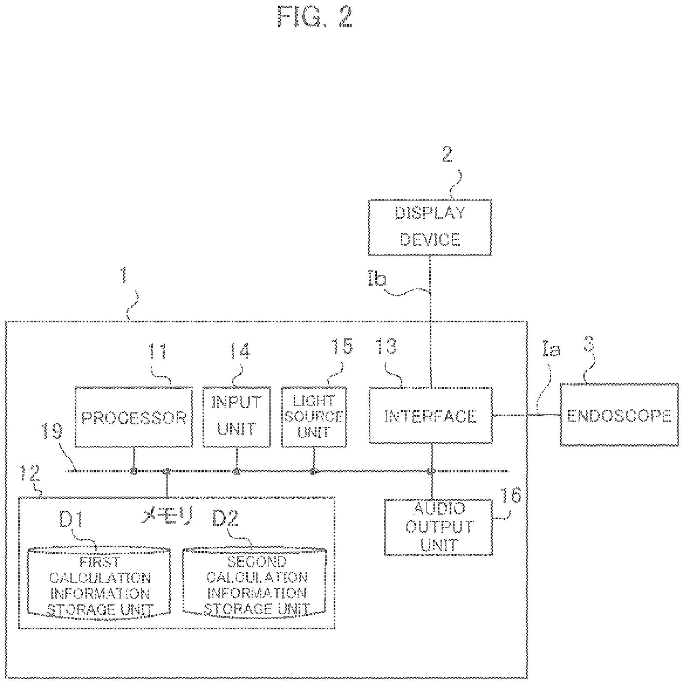

FIG. 2 shows the hardware configuration of the image processing device 1. The image processing device 1 mainly includes a processor 11, a memory 12, an interface 13, an input unit 14, a light source unit 15, and an audio output unit 16. Each of these elements is connected via a data bus 19.

The processor 11 executes a predetermined process by executing a program or the like stored in the memory 12. The processor 11 is one or more processors such as a CPU (Central Processing Unit), a GPU (Graphics Processing Unit), and a TPU (Tensor Processing Unit). The processor 11 may be configured by a plurality of processors. The processor 11 is an example of a computer.

The memory 12 is configured by a variety of volatile memories which are used as working memories, and nonvolatile memories which store information necessary for the process to be executed by the image processing device 1, such as a RAM (Random Access Memory) and a ROM (Read Only Memory). The memory 12 may include an external storage device such as a hard disk connected to or built in to the image processing device 1, or may include a storage medium such as a removable flash memory. The memory 12 stores a program for the image processing device 1 to execute each process in the present example embodiment.

The memory 12 functionally includes a first calculation information storage unit D1 for storing first calculation information and a second calculation information storage unit D2 for storing second calculation information. The first calculation information and the second calculation information are information used by the image processing device 1 in the classification regarding the presence or absence of a lesion part or information indicating the results of the calculation relating to the classification, and the details thereof will be described later. In addition, the memory 12 stores various parameters necessary for calculating the score of the classification regarding the existence of a lesion part. At least a portion of the information stored in the memory 12 may be stored in an external device other than the image processing device 1. In this case, the above-described external device may be one or more server devices capable of data communication with the image processing device 1 through a communication network or through direct communication.

The interface 13 performs an interface operation between the image processing device 1 and an external device. For example, the interface 13 supplies the display information "Ib" generated by the processor 11 to the display device 2. Further, the interface 13 supplies the light generated by the light source unit 15 to the endoscope 3. The interface 13 also provides an electrical signal to the processor 11 indicative of the captured image Ia supplied from the endoscope 3. The interface 13 may be a communication interface, such as a network adapter, for wired or wireless communication with the external device, or a hardware interface compliant with a USB (Universal Serial Bus), a SATA (Serial AT Attachment), or the like.

The input unit 14 generates an input signal based on the operation by the examiner. Examples of the input unit 14 include a button, a touch panel, a remote controller, and a voice input device. The light source unit 15 generates light for supplying to the tip unit 38 of the endoscope 3. The light source unit 15 may also incorporate a pump or the like for delivering water and air to be supplied to the endoscope 3. The audio output unit 16 outputs a sound under the control of the processor 11.

(3) Outline of Lesion part Detection Process

Next, an outline of the process of detecting a lesion part by the image processing device 1 will be described. In summary, the image processing device 1 detects the lesion part based on a variable number of time series captured images Ia. Thus, the image processing device 1 detects the lesion part accurately even for such a lesion part which is difficult to be identified from a single image, and presents the detection result. Furthermore, the image processing device 1 has a reset mechanism of the score according to the situation. Thus, even when the time series captured images Ia include images by which determination results of the lesion detection are different, or, when the time series captured images Ia include inappropriate images for the identification of the lesion, the image processing device 1 promptly detects a lesion part with computationally less burden. It is noted that examples of lesion parts difficult to be identified from a single image include a flat cancerized portion in Barrett esophagus or the like to be discriminated from a non-cancerized portion and a flat or the like.

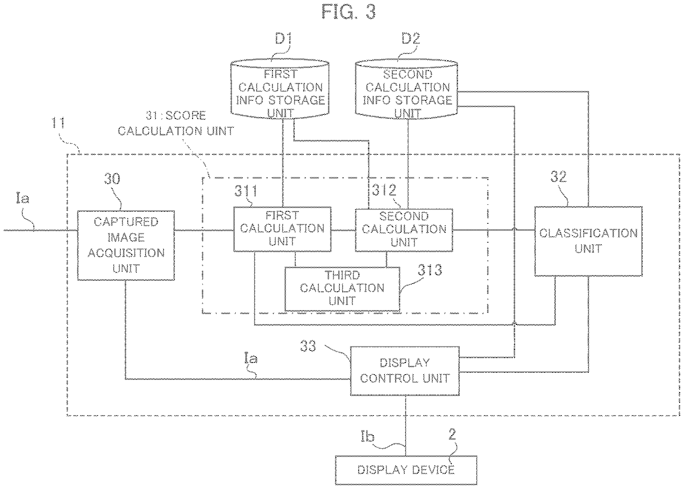

FIG. 3 is a functional block diagram of the image processing device 1. As shown in FIG. 3, the processor 11 of the image processing device 1 functionally includes a captured image acquisition unit 30, a score calculation unit 31, a classification unit 32, and a display control unit 33. In FIG. 3, blocks to transmit and receive data to or from each other are connected by a solid line, but the combination of blocks to transmit and receive data to or from each other is not limited to FIG. 3. The same applies to the drawings of other functional blocks described below.

The captured image acquisition unit 30 acquires the captured image Ia captured by the endoscope 3 through the interface 13 at predetermined intervals in accordance with the frame cycle of the endoscope 3. Then, the captured image acquisition unit 30 supplies the acquired captured image Ia to the score calculation unit 31 and the display control unit 33. Then, the score calculation unit 31, the classification unit 32, and the display control unit 33 performs the cyclic process described later at time intervals at which the captured image acquisition unit 30 acquires the captured image Ia. Hereafter, the timing of each cyclic process is also referred to as "processing time".

For each processing time, the score calculation unit 31 calculates a score (also referred to as "classification score") which indicates the likelihood of a presence of a lesion part, wherein the classification score is used for classification regarding the presence or absence of the lesion part. According to the present example embodiment, the score calculation unit 31 determines a classification score based on a likelihood ratio regarding time series captured images Ia. Here, the "likelihood ratio regarding time series captured images Ia" refers to the ratio between the likelihood that there is a lesion part in the time series captured images Ia and the likelihood that there is no lesion part in the time series captured images Ta. In the present example embodiment, as an example, it is assumed that the likelihood ratio increases with an increase in the likelihood that there is a lesion part.

The score calculation unit 31 functionally includes a first calculation unit 311, a second calculation unit 312, and a third calculation unit 313.

At each processing time, the first calculation unit 311 calculates a likelihood ratio regarding the latest "N" (N is an integral number) captured images Ia and supplies the calculation result to the second calculation unit 312. In this case, for example, the first calculation unit 311 calculates a likelihood ratio using a likelihood ratio calculation model, wherein the likelihood ratio calculation model is trained to output, when N captured images Ia are inputted thereto, a likelihood ratio regarding the inputted N captured images Ia. The likelihood ratio calculation model may be a deep learning model, or any other machine learning model or a statistical model. In this case, for example, learned parameters of the likelihood ratio calculation model are stored in the memory 12, and the first calculation unit 311 inputs the newest N captured images Ia to the likelihood ratio calculation model configured by referring to the learned parameters and acquires the likelihood ratio outputted by the model. If the likelihood ratio calculation model is constituted by a neural network, various parameters such as a layer structure, a neuron structure of each layer, the number of filters and filter sizes in each layer, and a weight for each element of each filter are previously stored in the memory 12. Even when the number of acquired captured images Ia is less than N, the first calculation unit 311 can acquire the likelihood ratio using the likelihood ratio calculation model from a less-than-N number of the captured images Ia.

The likelihood ratio calculation model may include an arbitrary feature extractor for extracting features (i.e., feature vector) of each captured image Ia that is inputted into the likelihood ratio calculation model, or may be configured separately from the feature extractor. In the latter case, the likelihood ratio calculation model is a model trained to output, when features of N captured images Ia extracted by the feature extractor are inputted to the model, a likelihood ratio relating to the N captured images Ia. In some embodiments, the feature extractor may extract the features representing the relation among time series data based on any technique for calculating the relation among time series data such as LSTM (Long Short Term Memory).

The first calculation unit 311 stores the likelihood ratio calculated at each processing time and data used by the first calculation unit 311 to calculate the likelihood ratio in the first calculation information storage unit D1 as the first calculation information. The "data used to calculate the likelihood ratio" may be captured images Ia used for calculation of the likelihood ratio, or may be the features extracted from the captured images Ia.

The second calculation unit 312 calculates a likelihood ratio (also referred to as "integrated likelihood ratio") into which the likelihood ratios calculated at the current processing time and past processing time(s) are integrated, and determines the classification score based on the integrated likelihood ratio. The classification score may be the integrated likelihood ratio itself or may be a value of a function including the integrated likelihood ratio as a variable.

It is hereinafter assumed that the time index "1" is assigned to a start time, the time index "t" is assigned to the current processing time, and "$x_i$" (i = 1, ..., t) stands for any captured image to be processed or its features. The term "start time" herein indicates the first processing time selected from the past processing times to be considered in the calculation of the classification score. In this instance, the integrated likelihood ratio for the binary classification between the class "$C_1$" which indicates the presence of a lesion part in captured images Ia and the class "$C_0$" which indicates the absence of a lesion part in captured images Ia is expressed by the following equation (1).

[Math Expression 1]

$$\log\left[\frac{p(x_1, \ldots, x_t | C_1)}{p(x_1, \ldots, x_t | C_0)}\right]$$
$$= \sum_{s=N+1}^{t} \log\left[\frac{p(C_1 | x_s, \ldots, x_{s-N})}{p(C_0 | x_s, \ldots, x_{s-N})}\right] - \sum_{s=N+2}^{t} \log\left[\frac{p(C_1 | x_{s-1}, \ldots, x_{s-N})}{p(C_0 | x_{s-1}, \ldots, x_{s-N})}\right]$$

Here, "p" stands for the probability belonging to each class (i.e., confidence level ranging from 0 to 1). In calculating the term on the right side of the equation (1), it is possible to use the likelihood ratio that the first calculation unit 311 stores as the first calculation information in the first calculation information storage unit D1.

Regarding the equation (1), since the time index t representing the current process time increases with the elapse of time, the length of the time series captured images Ia (or the features thereof) used for calculating the integrated likelihood ratio is a variable length. Thus, by using the integrated likelihood ratio based on the equation (1), the second calculation unit 312 can compute the classification score while considering a variable number of captured images Ia, as a first advantage. In addition, by using the integrated likelihood ratio based on the equation (1), it is possible to classify the time-dependent features as the second advantage. As the third advantage, the classification score allowing for a robust classification can be suitably calculated even when difficult-to-identify data is used. The second calculation unit 312 stores the integrated likelihood ratio and the classification scores computed at the respective processing times as the second calculation information in the second calculation information storage unit D2.

The third calculation unit 313 calculates a score (also referred to as "variation score") representing a degree of variation between a captured image Ia (also referred to as "current processing image") at the time index t representing the current processing time and a captured image Ia (also referred to as "past image") acquired at the immediately preceding time (i.e., the time index "t - 1"). The variation score increases with an increase in the degree of variation between the current processing image and the past image. Then, the third calculation unit 313 resets the classification score in the current calculation if the variation score is equal to or larger than a predetermined threshold value. In other words, the third calculation unit 313 stops calculation of the classification score in the current calculation, and starts calculation of the classification score obtained by setting the processing time after the change as the start time (i.e., the time index "1"). In this case, the first calculation unit 311 and the second calculation unit 312 of the score calculation unit 31 calculate the classification score based on the captured images Ia newly generated from the time when the reset is performed.

Here, a specific example of a method of calculating the variation score will be described.

In the first example, the third calculation unit 313 calculates, as the variation score, any index representing the degree of similarity based on comparison between images. Examples of the index representing the degree of similarity in this case include the correlation coefficient, SSIM (Structural SIMilarity), PSNR (Peak Signal-to-Noise Ratio), and the square errors between corresponding pixels.

In the second example, the third calculation unit 313 calculates the variation score based on the confidence level that a lesion part exists in the current processing image and the confidence level that a lesion part exists in the past image. For example, for each of the current processing image and the past image, the third calculation unit 313 generates a vector representing the confidence level that a lesion part exists in the image and the confidence level that any lesion part does not exist in the image. Then, the third calculation unit 313 calculates the variation score based on the inner product of the vectors. In this case, the third calculation unit 313 may calculate the above-described confidence level using a learned lesion detection model, wherein the lesion detection model is trained to output, when a captured image Ia is inputted to the model, a confidence level relating to the presence or absence of a lesion part in the input captured image Ia. In this case, for example, the lesion detection model is a machine learning model such as a neural network and a support vector machine, and the learned parameters are previously stored in the memory 12. The third calculation unit 313 may calculate the above-described variation score on the basis of the likelihood ratio calculated from the current processing image by the likelihood ratio calculation model and the likelihood ratio calculated from the past image by the likelihood ratio calculation model, instead of using the lesion detection model.

Next, a supplementary description will be given of the effect of the process executed by the third calculation unit 313. Generally, approximately thirty endoscopic images are taken per second, and since the movement of the endoscope between frames is basically slow, the variation in the photographed image Ia between frames becomes basically small. On the other hand, when the endoscope is moved quickly, when the camera shake by the examiner occurs, or when water is splashed on the examination point, etc., blurring in the captured image or the switchover of presence or absence of a lesion part in the captured image occurs and therefore the variation between frames becomes large. In view of the above, the third calculation unit 313 resets the classification score when the variation between frames is large, which allows for suitably suppressing delay of the classification determination timing in the classification unit 32 due to the above-described variation or an increase in the calculation load thereof.

The classification unit 32 performs classification of the presence or absence of the lesion part, based on the classification score calculated by the second calculation unit 312, and supplies the classification result to the display control unit 33. In this case, the classification unit 32 performs the classification described above using a threshold value of the classification score for determining the existence of a lesion part. In the present example embodiment, as an example, it is assumed that the higher the classification score is, the more likely a lesion part is to exist. Then, if the classification score is equal to or more than a predetermined threshold value (also referred to as "first threshold value Th1"), the classification unit 32 determines that a lesion part exists in the captured images Ia used for calculating the classification score. On the other hand, if the classification score is lower than a predetermined threshold value (also referred to as "second threshold value Th2") which is lower than the first threshold value, the classification unit 32 determines that there is no lesion part in the captured images Ia used for calculation of the classification score. For example, the first threshold value Th1 and the second threshold value Th2 are calibration values determined through experimental trials or the like, and are stored in advance in the memory 12 or the like.

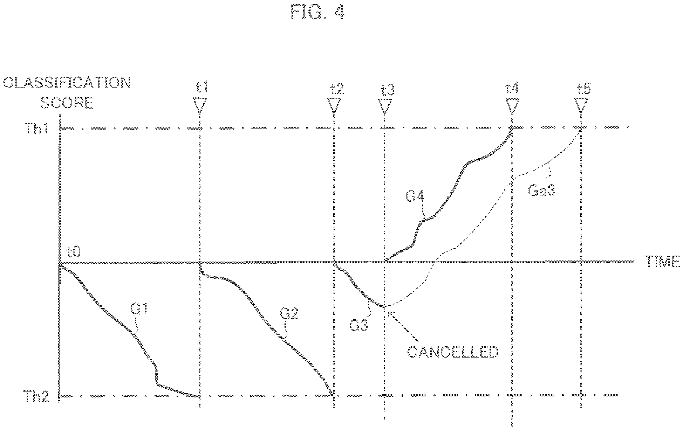

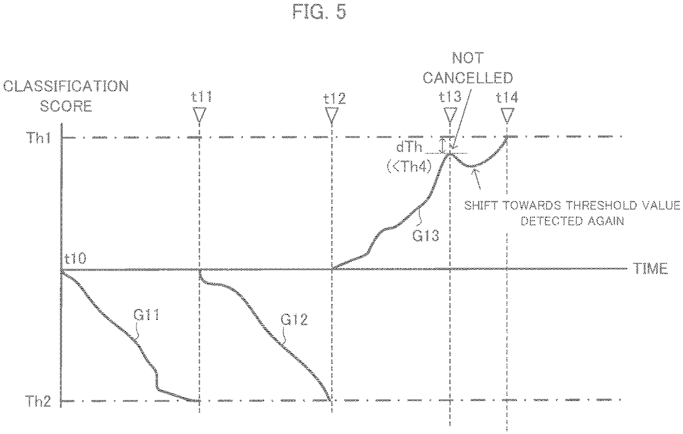

Then, the classification unit 32 determines the class if the classification score is equal to or larger than the first threshold value Th1 or equal to or smaller than the second threshold value Th2, and then notifies the score calculation unit 31 of information to the effect that the class is determined. In this case, the first calculation unit 311 and the second calculation unit 312 of the score calculation unit 31 reset the classification score and calculate the classification score based on the captured image Ia newly generated at or after the time of the notification. Specific examples of this process will be described in detail with reference to FIGS. 4 and 5. On the other hand, as long as the classification score does not reach any of these threshold values, the classification unit 32 does not make the determination of the class and keeps the class for the captured images Ia of interest pending (undetermined).

In some embodiments, upon determining that a predetermined condition other than the condition based on the first threshold value Th1 or the second threshold value Th2 is satisfied, the classification unit 32 may determine the class. For example, upon determining that the time index t representing the current processing time becomes equal to or larger than a predetermined threshold value, the classification unit 32 may determine the class. In this case, upon determining that the time index t representing the current processing time becomes equal to or larger than the predetermined threshold value, the classification unit 32 determines which of the first threshold value Th1 or the second threshold value Th2 is closer to the classification score at the time index t. Then, upon determining that the classification score is closer to the first threshold value Th1, the classification unit 32 determines that there is a lesion part. In contrast, upon determining that the classification score is closer to the second threshold value Th2, the classification unit 32 determines that there is no lesion part.

The display control unit 33 generates the display information Ib based on the captured image Ia and the classification result supplied from the classification unit 32. Then, by supplying the display information Ib to the display device 2 via the interface 13, the display control unit 33 causes the display device 2 to display the captured image Ia and information regarding the classification result by the classification unit 32. In some embodiments, the display control unit 33 may further cause the display device 2 to display information on the classification score stored in the second calculation information storage unit D2. The display example of the display control unit 33 will be described later.

Each component of the captured image acquisition unit 30, the score calculation unit 31, the classification unit 32, and the display control unit 33 can be realized, for example, by the processor 11 which executes a program. In addition, the necessary program may be recorded in any non-volatile storage medium and installed as necessary to realize the respective components. In addition, at least a part of these components is not limited to being realized by a software program and may be realized by any combination of hardware, firmware, and software. At least some of these components may also be implemented using user-programmable integrated circuitry, such as FPGA (Field-Programmable Gate Array) and microcontrollers. In this case, the integrated circuit may be used to realize a program for configuring each of the above-described components. Further, at least a part of the components may be configured by a ASSP (Application Specific Standard Produce), ASIC (Application Specific Integrated Circuit) and/or a quantum processor (quantum computer control chip). In this way, each component may be implemented by a variety of hardware. The above is true for other example embodiments to be described later. Further, each of these components may be realized by the collaboration of a plurality of computers, for example, using cloud computing technology.

(4) Specific Embodiment of Lesion part Detection Process

Next, a specific embodiment of the process of calculating the classification score will be described. The image processing device 1 executes either a sequential calculation process or a parallel calculation process, wherein the sequential calculation process is a process of updating the start time and sequentially calculating the classification score based on the updated start time and the parallel calculation process is a process of setting the start time at predetermined time intervals and calculating the classification scores in parallel for respective set start times. Hereinafter, a specific example of the sequential calculation process will be described, and then a specific example of the parallel calculation process will be described.

(4-1) Sequential Calculation Process

FIG. 4 is a graph showing the transition of the classification score calculated based on the sequential calculation process. In this example, the image processing device 1 starts processing from the time "t0" and calculates the classification score based on the captured images Ia obtained at intervals of the frame cycle of the endoscope 3. Then, after resetting the classification score at the time "t1", the time "t2", and the time "t3", the image processing device 1 generates the classification result to the effect that the lesion part exists at the time "t4".

First, during the period from the time t0 to the time t1, since any captured image Ia including a lesion part is not generated, as shown in the graph G1, the classification score gradually approaches the second threshold value Th2. Since the classification score does not reach any of the threshold values until the time t1, the classification unit 32 holds off the decision of the classification for the captured images Ia and keeps the class undetermined. In this case, the classification unit 32 may supply information to the effect that the class is undetermined to the display control unit 33.

In addition, after the time t0, the score calculation unit 31 executes a process of determining, at every processing time, whether or not the variation score representing the degree of variation between the current processing image and the past image becomes equal to or larger than a predetermined threshold value (also referred to as "third threshold value Th3"). As will be described later, the variation score becomes equal to or larger than the third threshold value Th3 at time t3, and has been less than the third threshold value Th3 until then. As long as the variation score is less than the third threshold value Th3, the score calculation unit 31 does not perform the updating of the start time based on the variation score.

Then, at the time t1, the classification score calculated based on the captured images Ia obtained during the period from the time t0 to the time t1 becomes equal to or less than the second threshold value Th2. Therefore, in this case, the classification unit 32 supplies the display control unit 33 with the classification result to the effect that any captured image Ia obtained from the time t0 to the time t1 does not include a lesion part, and supplies the score calculation unit 31 with the notification for resetting the calculation process of the classification score (i.e., updating the start time).

Then, the score calculation unit 31 resets the classification score on the basis of the notification to the effect that the class is determined supplied from the classification unit 32 at the time t1. Then, the score calculation unit 31 updates the start time in computing the classification score to the time t1 and starts computing the classification score for the captured images Ia obtained after the time t1. Thereafter, as shown in the graph G2, at the time t2, the classification score calculated by the score calculation unit 31 based on the captured images Ia obtained during the period from the time t1 to the time t2 is equal to or less than the second threshold value Th2. Therefore, in this case, the classification unit 32 generates a classification result to the effect that any lesion part is not included in the captured images Ia obtained during the period from the time t1 to the time t2. Then, the classification unit 32 supplies the generated classification result to the display control unit 33, and supplies a notification for resetting the calculation process of the classification score to the score calculation unit 31. Then, on the basis of the notification to the effect that the classification has been performed by the classification unit 32 at the time t2, the score calculation unit 31 updates the start time in computing the classification score to be the time t2, and starts computing the classification score targeting the captured images Ia obtained after the time t2 (see the graph G3).

Thereafter, at the time t3, the score calculation unit 31 determines that the variation score representing the degree of variation between the current processing image and the past image has become the third threshold value Th3 or more, and therefore updates the start time and resets the classification score. At the time t3, a captured image Ia including a lesion part is obtained (i.e., the change in the classes has occurred), and the classification score has started to rise.

In this instance, the score calculation unit 31 stops the calculation process of the present classification score and starts the calculation process of the classification score in which the start calculation time of the classification score is updated to be the processing time subsequent to the time t3 (see the graph G4). Thereafter, the classification score calculated by the score calculation unit 31 based on the captured images Ia obtained until the time "t4" becomes equal to or larger than the first threshold value Th1. Therefore, in this case, the classification unit 32 generates a classification result to the effect that a lesion part is included in each captured image Ia obtained during the period from the subsequent processing time of the time t3 to the time t4. In this way, the image processing device 1 resets the classification score at the time t3, which allows for prompt determination that a lesion part is included.

On the other hand, if the resetting of the classification score based on the variation score at the time t3 were not performed, the classification score in consideration of the captured images Ia without any lesion part obtained from the time t2 to the time t3 would be calculated continuously even after the time t3. Thus, in that case, as shown in the graph Ga3, the time when the classification score is equal to or more than the first threshold value Th1 would become the time t5, which is later than the time t4. If the lesion part fell outside the imaging range of the endoscope 3 during the period from the time t4 to the time t5, the classification score would not reach the first threshold value Th1.

Thus, not only in the case where the classification score becomes equal to or larger than the first threshold value Th1 or smaller than the second threshold value Th2 (i.e., when the determination result of the classification comes out), but also in the case where the variation score becomes equal to or larger than the third threshold value Th3, the score calculation unit 31 updates the start time and resets the classification score. Thus, the score calculation unit 31 can suitably suppress the delay of the classification determination timing and an increase in the computational burden in the classification unit 32 due to the occurrence of the switching between the presence an the absence of a lesion part in the captured image Ia or the generation of a blurred captured image Ia.

Resetting the classification score based on the variation score in a condition where the classification score is about to reach the first threshold value Th1 or the second threshold value Th2 might cause the delay of the classification determination timing. In view of the above, even if the variation score is equal to or larger than the third threshold value Th3, the score calculation unit 31 may cancel the process of resetting the classification score as long as the difference (also referred to as "threshold difference dTh") between the classification score and either the first threshold value Th1 or the second threshold value Th2, whichever comes closer to the classification score, is less than a predetermined threshold value (also referred to as "fourth threshold value Th4"). Then, upon determining that the classification score has shifted towards a direction (i.e., a direction approaching the threshold value) in which the threshold difference dTh decreases within a predetermined time (e.g., several frames), the score calculation unit 31 finally determines the cancellation of the process of resetting the classification score, and does not update the start time. On the other hand, upon determining that the classification score has not shifted towards the direction in which the threshold difference dTh decreases, the score calculation unit 31 executes a process of resetting the classification score and updates the start time. In this instance, the score calculation unit 31 calculates the derivative of the classification score and determines, based on the calculated derivative value, whether or not the classification score has shifted towards the direction in which the threshold difference dTh decreases.

Thus, the score calculation unit 31 can suitably suppress the delay of the classification determination timing due to the update of the start time of the classification score in a condition where the classification score is about to reach the first threshold value Th1 or the second threshold value Th2.

FIG. 5 is a graph illustrating a transition of the classification score for determining the necessity of the update of the start time in consideration of the threshold difference dTh. In this example, the image processing device 1 outputs the classification result, which indicates the absence of any lesion part, regarding the captured images Ia during the period from the time "t10" to the time "t11" and the period from the time t11 to the time "t12" while outputting the classification result, which indicates the presence of a lesion part, regarding the captured images Ia during the period from the time t12 to the time "t14".

In this example, at the time "t3" which is the middle of the calculation process of the classification score in which the time 112 is the start time, the score calculation unit 31 determines that the variation score is equal to or larger than the third threshold value Th3. Therefore, in this case, the score calculation unit 31 calculates the threshold difference dTh which is the difference between the classification score at the time t3 and either the first threshold value Th1 or the second threshold value Th2 whichever (in this case the first threshold value Th1) closer to the classification score and then determines whether the threshold difference dTh is less than the fourth threshold value Th4. Then, since the threshold difference dTh is less than the fourth threshold value Th4, the score calculation unit 31 updates the classification score during a period equivalent to a predetermined number of frames from the time t3, and then monitors whether or not the classification score has shifted towards the direction in which the threshold difference dTh decreases. Then, since the score calculation unit 31 detects that the classification score has shifted in the direction in which the threshold difference dTh decreases during the monitoring period, the score calculation unit 31 cancels the reset of the classification score, and continues the calculation process of the classification score with the time t12 as the start time. Thereafter, since the classification score at the time t14 is equal to or larger than the first threshold value Th1, the classification unit 32 outputs the classification result to the effect that a lesion part is included. Thus, by using the threshold difference dTh, the image processing device 1 can suppress the delay of the classification determination timing due to the resetting of the classification score based on the variation score.

(4-2) Parallel Calculation Process

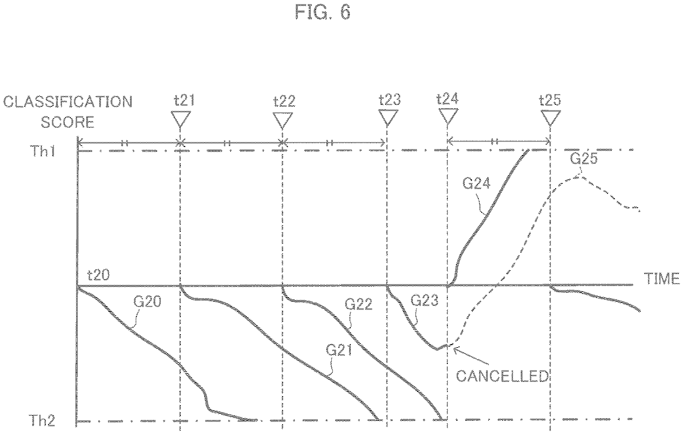

FIG. 6 is a graph showing the transition of the classification score calculated based on the parallel calculation process. In this example, the image processing device 1 starts processing from the time "t20" and calculates the classification score based on the captured images Ia obtained according to the frame cycle of the endoscope 3. Then, the image processing device 1 starts the calculation processes of the classification scores in which the times "t21", "t22", "t23", "t24", and "t25" are set as the start times, respectively.

In this example, at predetermined time intervals from the time t20, the score calculation unit 31 repeatedly sets the start time of the captured image Ia used for calculating the classification score and starts a thread (process flow) for computing the classification score whenever the start time is set. Here, the term "predetermined time intervals" indicates, for example, intervals corresponding to integral multiple of the frame cycle, and is specifically set to time intervals (e.g., time intervals which allow for the real-time processing) according to the processing capacity or the like of the image processing device 1.

In each thread, a calculation of the classification score is continuously performed until the classification score reaches either the first threshold value Th1 or the second threshold value Th2 or until the variation score becomes equal to or larger than the third threshold value Th3. As a result, the calculations of the classification scores with different start times are performed in parallel. For example, during the period from the time t21 to the time t22, there is a thread (see the graph G20) that starts at the time t20 and a thread (see the graph G21) that starts at the time t21.

Then, at around the time t24 at which a lesion part is photographed, a thread (see the graphical G23) which started at the time t23 has been active. In the thread which starts at the time t23, the classification score approaches the second threshold value Th2 during the period from the time t23 to the time t24.

At the time t24, the score calculation unit 31 determines that the variation score is equal to or larger than the third threshold value Th3 and resets the classification score. At the time t24, captured images Ia in which a lesion part is photographed are obtained (that is, the variation in the class has occurred), and the classification score is rising. In this instance, the score calculation unit 31 cancels the current calculation process of the classification score and starts a new calculation process of the classification score which starts at the processing time immediately after the time t24 (see the graph G24). Subsequently, since the classification score calculated by the score calculation unit 31 becomes larger than the first threshold value Th1, the classification unit 32 generates a classification result to the effect that a lesion part exists. In this way, the image processing device 1 resets the classification score at the time t24, which allows for a prompt detection of a lesion part. Thereafter, the score calculation unit 31 starts a thread which starts at the time "t25" after a predetermined time interval from the time t24 which is the previous start time.

On the other hand, if the classification score were not reset based on the variation score at the time t24, the classification score which has been calculated from the time t23 as the start time, would transition as shown in the graph G25 after the time t24. In this situation, since any lesion part does not exist in the captured images Ia after the time t25, the classification score would decrease again prior to reaching the first threshold value Th1. In this case, it is not possible to obtain a classification result to the effect that a lesion part exists.

As described above, even in the parallel calculation process, the score calculation unit 31 resets the classification score when the variation score becomes equal to or larger than the third threshold value Th3. This allows for suitable suppression of the delay of the classification determination timing and the increase in the calculation load in the classification unit 32 caused by the fluctuation in the captured image Ia.

In some embodiments, in the parallel calculation process, in the same way as in the sequential calculation process, even if the variation score becomes the third threshold value Th3 or more, the image processing device 1 cancels the reset of the classification score and monitors whether or not the threshold difference dTh decreases as long as the threshold difference dTh is smaller than the fourth threshold value Th4. In this case, if the classification score has shifted in a direction in which the threshold difference dTh decreases within a predetermined time, the image processing device 1 does not reset the classification score. In contrast, if the classification score has not shifted in a direction in which the threshold difference dTh decreases within the predetermined time, the image processing device 1 resets the classification score.

Here, a supplementary description will be given of the effect of resetting the classification score based on the variation score in the parallel calculation process. If the classification score is not reset based on the variation score in the parallel calculation process, the above-described blur or the switchover of the presence or absence of a lesion part in the captured image Ia could leads to the situation where the classification score does not reach the threshold value or the classification error occurs. On the other hand, if the time interval for starting a thread is sufficiently shortened, the computational load becomes high although the classification score is less likely to reach the threshold value or cause misclassification. In view of the above, the image processing device 1 resets the classification score based on the variation score in the parallel calculation process. Thus, while reducing the computational burden, it is possible to suitably reduce the possibility that the classification score does not reach the threshold value or a classification error occurs.

(5) Display Examples

Next, a description will be given of the display control of the display device 2 to be executed by the display control unit 33.

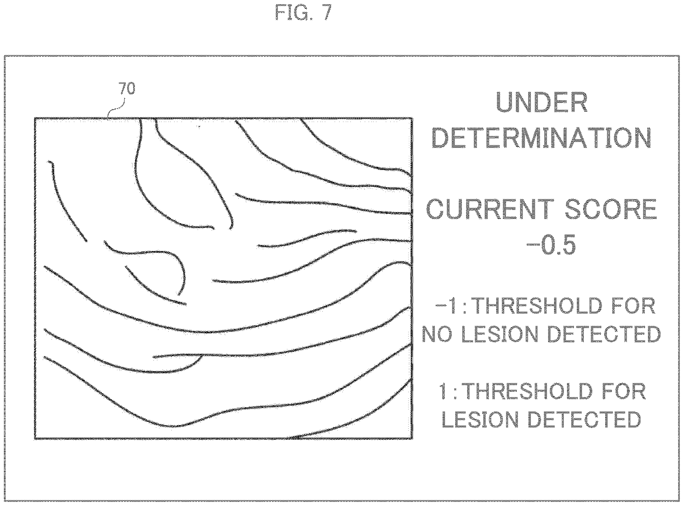

FIG. 7 shows a first display example of a display screen image displayed by the display device 2 in endoscopic examination. The display control unit 33 of the image processing device 1 outputs display information Ib generated based on the classification result generated by the classification unit 32 and the captured image Ia acquired by the captured image acquisition unit 30 to the display device 2. The display control unit 33 transmits the captured image Ia and the display information Ib to the display device 2 to cause the display device 2 to display the display screen image shown in FIG. 7.

In the first display example, the display control unit 33 of the image processing device 1 provides the latest image display area 70, which represents the latest captured image Ia captured by the captured image acquisition unit 30, while displaying information regarding the lesion part on the display screen image. Here, the score calculation unit 31 performs the sequential calculation process, and the most recent classification score calculated by the score calculation unit 31 does not reach any of the first threshold value Th1 and the second threshold value Th2. As a result, the classification unit 32 has not been able to determine the class. Therefore, through the classification score or the notification from the classification unit 32, the display control unit 33 recognizes that the classification unit 32 has not been able to determine the class, and therefore displays the statement to the effect that the class regarding the presence or absence of a lesion part is not determination (i.e., under determination as to whether or not there is a lesion part) on the display screen image. In addition to the information described above, the display control unit 33 also displays the latest classification score (here -0.5) at the current time and the description of the threshold values on the display screen image.

Thus, in the first display example, when the class for the captured images Ia is undetermined, the display control unit 33 outputs information indicating that the classification is undetermined together with information regarding the classification score or the like. Thus, the display control unit 33 can suitably visualize the current state related to the existence determination of a lesion part.

In some embodiments, the display control unit 33 may display, together with the display of the current classification score, or in place of this, a graph representing the transition of the classification score on the display screen image. While the score calculation unit 31 performs the parallel calculation process, the display control unit 33 may display the respective classification scores corresponding to ongoing threads, may display the classification score closest to either the first threshold value Th1 or the second threshold value Th2, or may display a graph shown in FIG. 5 on the display screen image. If the class for the captured images Ia has not been determined, the display control unit 33 may instruct the audio output unit 16 to provide a voice guidance or a predetermined warning sound to the effect that the classification is undetermined, in addition to or in place of the display control shown in the first display example. Thereby, the display control unit 33 suitably enables the examiner to grasp that the classification is undetermined.

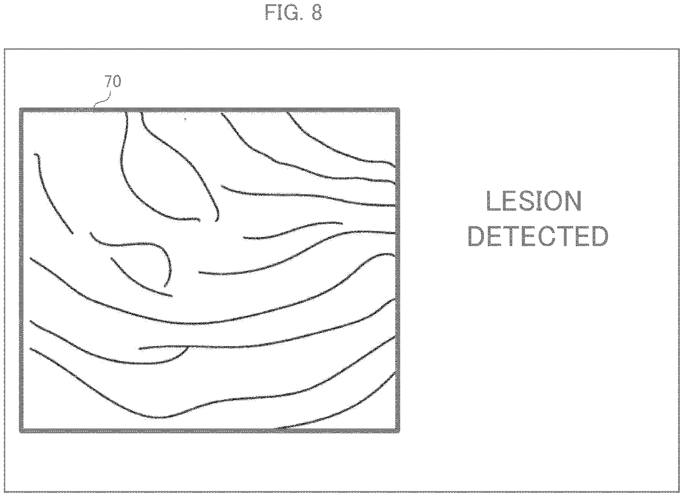

FIG. 8 shows a second display example of a display screen image displayed by the display device 2 in the endoscopic examination. In the second display example, the classification unit 32 determines that the classification score supplied from the score calculation unit 31 becomes equal to or larger than the first threshold value Th1, and supplies the classification result indicating the class of the presence of a lesion part to the display control unit 33. Then, in this case, in order to notify the examiner that the lesion part is present, the display control unit 33 highlights the latest image display area 70 by edging effect while displaying, on the display screen image, the statement to the effect that the lesion part has been detected.

Thus, in the second display example, if the captured image Ia is classified as the class of the presence of a lesion part, the display control unit 33 outputs information indicating the presence of the lesion part (in FIG. 7, the edging effect of the latest image display area 70 and the sentence). Thus, the display control unit 33 can suitably notify the examiner that the lesion part is detected.

If the classification score supplied from the score calculation unit 31 is equal to or less than the second threshold value Th2, the display control unit 33 notifies the examiner of the absence of any lesion part. Thus, the display control unit 33 can suitably prompt the examiner to move the point photographed by the endoscope 3 (to go ahead with the operation of the endoscope 3).

In some embodiments, if the classification score becomes the first threshold value Th1 or more, the display control unit 33 may instruct, instead of the display control shown in the second display example or in addition to this, the audio output unit 16 to output the voice guidance or a warning sound to the effect that a lesion part exists. Thereby, the display control unit 33 also enables the examiner to suitably grasp the presence of the lesion part. Similarly, once the classification score becomes equal to or less than the second threshold value Th2, the display control unit 33 may instruct the audio output unit 16 to output a voice or an alert sound notifying the examiner that any lesion part is not detected.

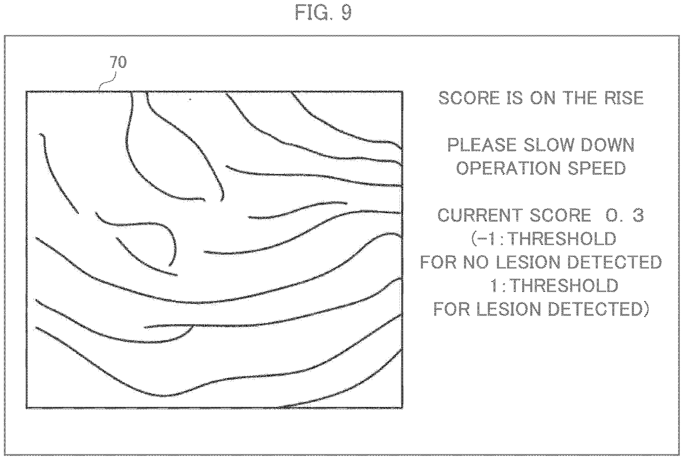

FIG. 9 shows a third display example of a display screen image displayed by the display device 2 in the endoscopic examination. In the third display example, the display control unit 33 monitors the transition of the classification score by referring to the second calculated information storage unit D2, and determines that the classification score is on the rise. Therefore, the display control unit 33 displays, on the display screen image together with the current classification score, statements to the effect that the classification score is on the rise and therefore the operation speed of the endoscope 3 should be slowed down (that is, the photographing target should be fixed as much as possible). In this case, for example, the display control unit 33 determines that the classification score is on the rise, upon determining that a predetermined number of latest classification scores are a predetermined rate or more higher than the classification scores each calculated at the immediately preceding processing time, or, the average of the predetermined number of latest classification scores is higher than the average of the predetermined number of classification scores calculated immediately before the latest classification scores. The determination of the rise in the classification score is not limited to the above-mentioned example, and any statistical method may be used to make the determination. In this way, the display control unit 33 may output information regarding the variation in the classification score. The display control unit 33 may output operation instructions of the endoscope 3 based on the information on the variation in the classification score.

Thus, according to the third display example, when the classification score is in the upward tendency, the display control unit 33 outputs information indicating that the classification score is in the upward tendency. Thus, the display control unit 33 notifies the examiner that the classification score is on the rise, which suitably suppresses a lesion part from not being detected due to the fluctuation in the photographed position in the middle of determination regarding the presence or absence of the lesion part. The display control unit 33 may instruct, instead of or in addition to the display control shown in the third display example, the audio output unit 16 to output the voice or warning sound notifying the examiner that the classification score is on the rise.

(6) Processing Flow

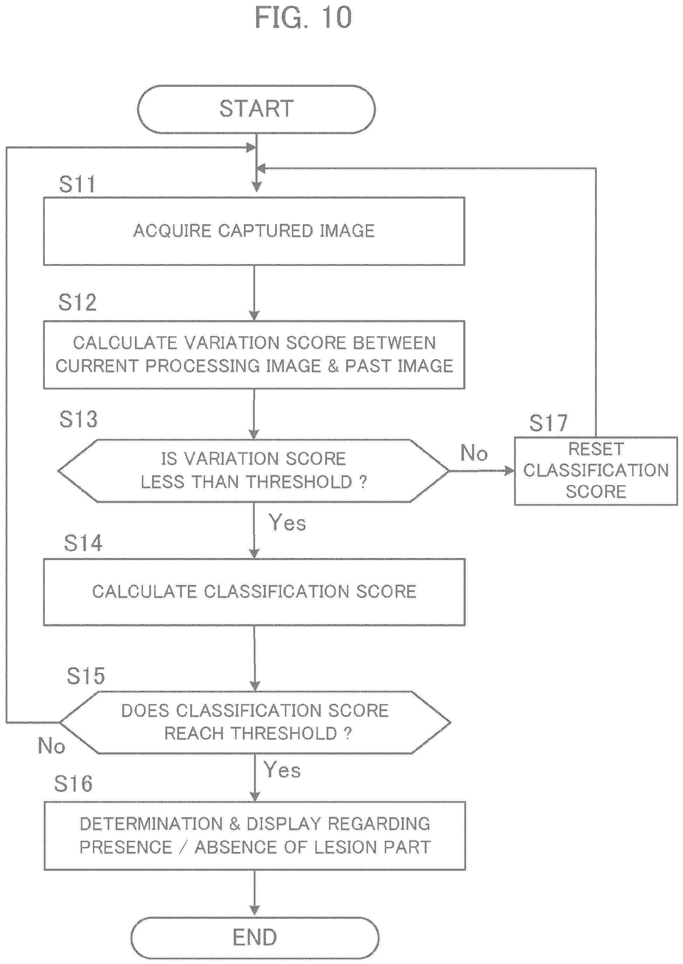

FIG. 10 is an example of a flowchart that is executed by the image processing device 1 with respect to detection of the lesion part based on the sequential calculation process. The image processing device 1 repeatedly executes processing of the flowchart until the end of the endoscopic examination. For example, upon detecting a predetermined input or the like to the input unit 14 or the operation unit 36, the image processing device 1 determines that the endoscopic examination has been completed.

First, the captured image acquisition unit 30 of the image processing device 1 acquires a captured image Ia (step S11). In this instance, the captured image acquisition unit 30 of the image processing device 1 receives a captured image Ia from the endoscope 3 via the interface 13. The display control unit 33 executes a process such as to display the captured image Ia acquired at step S11 on the display device 2.

Next, the score calculation unit 31 of the image processing device 1 calculates the variation score based on the current processing image that is the captured image Ia obtained at step S11 at the current processing time and the past image that is the captured image Ia obtained at the step S11 at the immediately preceding processing time (step S12). Then, the score calculation unit 31 determines whether or not the variation score is less than the third threshold value Th3 (step S13).

If the variation score is less than the third threshold value Th3 (step S13; Yes), the score calculation unit 31 calculates the classification score at the current process time (step S14). On the other hand, if the variation score is equal to or larger than the third threshold value Th3 (step S13; No), the score calculation unit 31 resets the classification score (step S17). In this case, the score calculation unit 31 cancels the calculation process regarding the current classification score and starts a new calculation process regarding the classification score which starts at the subsequent processing time. In some embodiments, if the threshold difference dTh is less than the fourth threshold value Th4, the score calculation unit 31 monitors whether or not the classification score shits in the direction in which the threshold difference dTh decreases. Then, the score calculation unit 31 executes the process at step S17 only in the condition that the classification score does not shift in the direction in which the threshold difference dTh decreases.

In calculating the classification score at step S14, first, the score calculation unit 31 acquires, as the first calculation information, captured images Ia acquired in the past in processing the flowchart, or the features thereof, and then calculates the likelihood ratio based on the first calculation information and the captured image Ia acquired at step S11 at the current processing time. The score calculation unit 31 stores the calculated likelihood ratio and the captured image Ia acquired at step S11 or the features thereof in the first calculation information storage unit D1 as the first calculation information. Then, the score calculation unit 31 calculates the integrated likelihood ratio based on the equation (1) by referring to the likelihood ratio or the like stored in the first calculation information storage unit D1, and determines that classification score is the calculated integrated likelihood ratio or the value of the function in which the integrated likelihood ratio is used as a variable. The score calculation unit 31 stores the calculated classification score or the like as the second calculation information in the second calculation information storage unit D2. The display control unit 33 may perform processing for displaying the classification score or the like calculated by the score calculation unit 31 on the display device 2.

Next, the classification unit 32 of the image processing device 1 determines whether or not the classification score has reached either the first threshold value Th1 or the second threshold value Th2 (step S15). Then, upon determining that the classification score has reached either one of the threshold values (step S15; Yes), the classification unit 32 makes a determination (classification) regarding the presence or absence of a lesion part. Furthermore, the display control unit 33 causes the display device 2 to display information based on the determination result (classification result) as to the presence or absence of the lesion part by the classification unit 32 (step S16). On the other hand, upon determining that the classification score has not reached any one of the first threshold value Th1 and the second threshold value Th2 (step S15; No), the classification unit 32 proceeds back to the process at step S11. In this case, the image processing device 1 updates the classification score by further considering the captured image Ia acquired at the subsequent processing time.

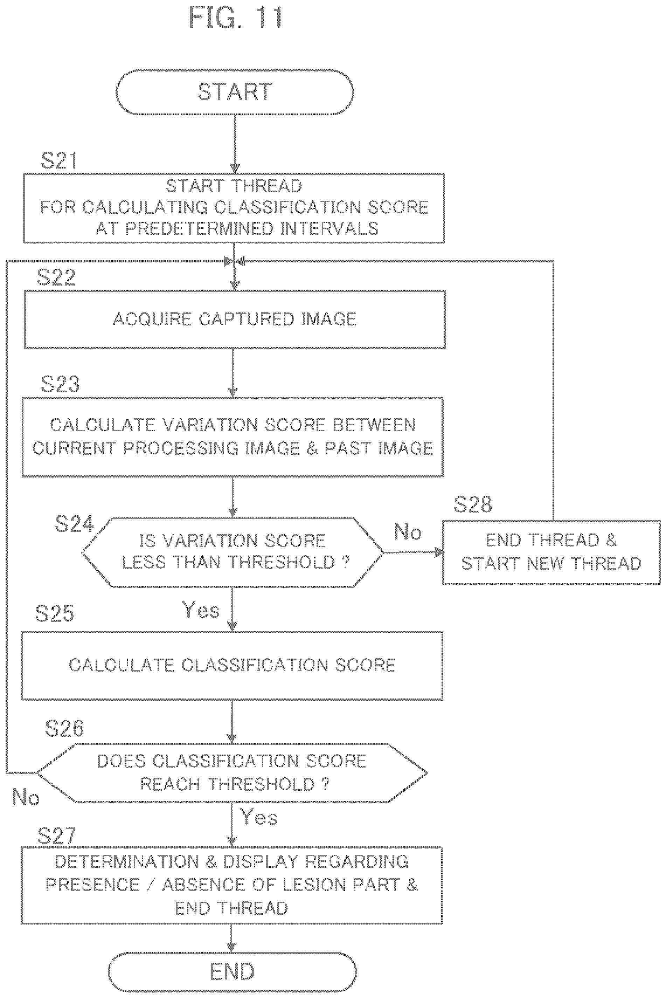

FIG. 11 is an example of a flowchart that is executed by the image processing device 1 with respect to detection of a lesion part based on parallel calculation process. The image processing device 1 repeatedly executes the process of the flowchart until the end of the endoscopic examination.

First, the image processing device 1 starts up a thread of the classification score calculation at predetermined time intervals (step S21). At step S22 to step S25, the image processing device 1 executes the same process as step S11 to step S14 in FIG. 10 in every ongoing thread. That is, the image processing device 1 acquires the captured image Ia (step S22), calculates the variation score (step S23), determines whether or not the variation score is less than the third threshold value Th3 (step S24), and calculates the classification score if the variation score is less than the third threshold value Th3 (step S25), respectively.

If the variation score is equal to or larger than the third threshold value Th3 (step S24; No), the score calculation unit 31 ends the ongoing thread and starts a new thread of the classification score calculation which starts at the current processing time or the subsequent processing time (step S28). Accordingly, the image processing device 1 reduces the influence of generation of blurring in the captured image Ia and/or the switchover between the presence and absence of a lesion part in the captured image Ia. Even in such a situation where the threshold difference dTh is less than the fourth threshold value Th4, the score calculation unit 31 may monitor whether or not the classification score shifts in the direction in which the threshold difference dTh decreases to perform the process at step S28 only when the classification score does not shift in the direction in which the threshold difference dTh decreases.

Then, the classification unit 32 of the image processing device 1 determines whether or not the classification score reaches either the first threshold value Th1 or the second threshold value Th2 after the calculation of the classification score at step S25 (step S26). Once the classification score reaches any one of the threshold values (step S26; Yes), the image processing device 1 determines (classifies) the presence or absence of the lesion part and displays information based on the determination result (classification result) as to the presence or absence of the lesion part by the classification unit 32 on the display device 2, and terminates the ongoing thread in the target flowchart (step S27). On the other hand, if the classification score reaches neither one of the first threshold value Th1 nor the second threshold value Th2 (step S26; No), the process goes back to step S22. In this instance, in the ongoing thread, the image processing device 1 updates the classification score by further considering the captured image Ia acquired at the subsequent processing time.

(7) Modifications

Next, a modification suitable for the above-described example embodiment will be described. The following modifications may be applied to the example embodiments described above in any combination.

(First Modification)

The image processing device 1 may process a video, which is configured by the captured images Ia generated during an endoscopic examination, after the examination.

For example, upon detecting that a video to be processed is designated based on the user input by the input unit 14 at any timing after the examination, the image processing device 1 repeatedly performs the process of the flowchart shown in FIG. 10 or FIG. 11 for the time series captured images Ia constituting the video until it is determined that the video reaches the end.

(Second Modification)

The target to be detected by the detection model is not limited to a lesion part, and may be any region of interest where the examiner needs attention. Examples of the region of interest include not only a lesion part but also a part with inflammation, a part with an operative scar or other cut part, a part with a fold or protrusion, and a part on the wall surface of the lumen where the tip unit 38 of the endoscope 3 tends to get contact (caught).

(Third Modification)

Instead of performing a binary classification as to the presence or absence of the lesion part, the classification unit 32 may perform a classification into three or more classes. For example, in this case, the classification unit 32 classifies images into X + 1 ("X" is an integer of 2 or more) classes: "image group with first lesion type" to "image group with Xth lesion type" and "image group without any lesion part".

In this case, for example, provided that the number of classes to be classified is M, the score calculation unit 31 calculates the integrated likelihood ratio between the kth class and all classes other than the kth class among the M classes. In this case, for example, the score calculation unit 31 calculates the integrated likelihood ratio while replacing the denominator of the first term and the second term on the right side of the equation (1) with the maximum likelihood among all the classes other than the kth class. In this case, instead of using the maximum likelihood, the score calculation unit 31 may calculate the integrated likelihood ratio using the sum of the likelihoods of all the classes other than the kth class.

Then, based on the classification result outputted by the classification unit 32, the display control unit 33 displays on the display device 2 information regarding the presence or absence of a lesion part and the lesion type when there is a lesion part. In this way, the image processing device 1 can suitably assist the examiner to make a diagnosis.

<Second Example Embodiment>

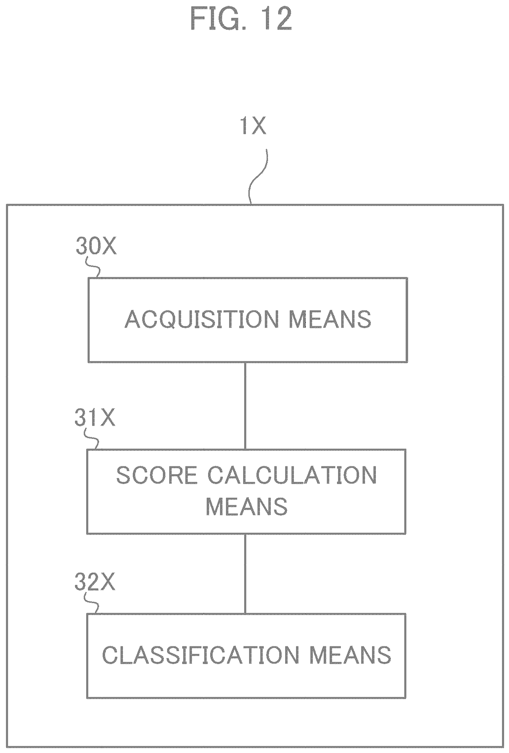

FIG. 12 is a block diagram of an image processing device 1X according to the second example embodiment. The image processing device 1X includes an acquisition means 30X, a score calculation means 31X, and a classification means 32X. The image processing device 1X may be configured by a plurality of devices.

The acquisition means 30X is configured to acquire a captured image obtained by photographing an examination target by a photographing unit provided in an endoscope. In this instance, the acquisition means 30X may immediately acquire the captured image generated by the photographing unit, or may acquire, at a predetermined timing, the captured image stored in the storage device generated in advance by the photographing unit. Examples of the acquisition means 30X include the captured image acquisition unit 30 in the first example embodiment (including modifications, hereinafter the same).

The score calculation means 31X is configured to calculate, based on the captured images in time series obtained from a start time, a score regarding a likelihood of a presence of a region of interest in the captured images. Here, upon detecting a variation in the captured images, the score calculation means 31X sets a time after the variation as the start time. Examples of the score calculation means 31X include the score calculation unit 31 in the first example embodiment.

The classification means 32X is configured to classify, based on the score, the captured images in time series if a predetermined condition is satisfied. Examples of the "predetermined condition" include a condition based on the score and a condition based on the number of captured images in time series. Examples of the classification means 32X include the classification unit 32 in the first example embodiment.

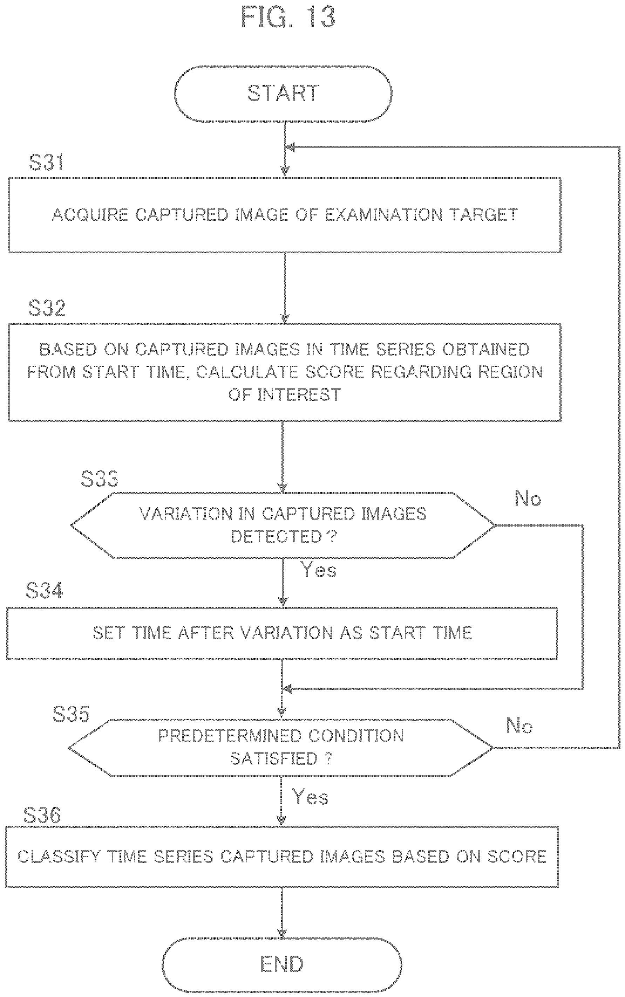

FIG. 13 is an example of a flowchart showing a processing procedure in the second example embodiment. First, the acquisition means 30X acquires a captured image obtained by photographing an examination target by a photographing unit provided in an endoscope (step S31). The score calculation means 31X calculates, based on the captured images in time series obtained from a start time, a score regarding a likelihood of a presence of a region of interest in the captured images (step S32). Then, upon detecting a variation in the captured images (step S33; Yes), the score calculation means 31X sets a time after the variation as the start time (step S34). If a variation in the captured images is not detected (step S33; No), the process at step S34 is not performed. Then, if a predetermined condition is satisfied (step S35; Yes), the classification means 32X classifies, based on the score, the captured images in time series (step S36). On the other hand, if the predetermined condition is not satisfied (step S35; No), the process gets back to step S31.

According to the second example embodiment, the image processing device 1X can accurately detect a region of interest on the basis of time-series captured images.

In the example embodiments described above, the program is stored by any type of a non-transitory computer-readable medium (non-transitory computer readable medium) and can be supplied to a control unit or the like that is a computer. The non-transitory computer-readable medium include any type of a tangible storage medium. Examples of the non-transitory computer readable medium include a magnetic storage medium (e.g., a flexible disk, a magnetic tape, a hard disk drive), a magnetic-optical storage medium (e.g., a magnetic optical disk), CD-ROM (Read Only Memory), CD-R, CD-R/W, a solid-state memory (e.g., a mask ROM, a PROM (Programmable ROM), an EPROM (Erasable PROM), a flash ROM, a RAM (Random Access Memory)). The program may also be provided to the computer by any type of a transitory computer readable medium. Examples of the transitory computer readable medium include an electrical signal, an optical signal, and an electromagnetic wave. The transitory computer readable medium can provide the program to the computer through a wired channel such as wires and optical fibers or a wireless channel.

The whole or a part of the example embodiments described above (including modifications, the same applies hereinafter) can be described as, but not limited to, the following Supplementary Notes.

[Supplementary Note 1]

An image processing device comprising:

an acquisition means configured to acquire a captured image obtained by photographing an examination target by a photographing unit provided in an endoscope;
a score calculation means configured to calculate, based on the captured images in time series obtained from a start time, a score regarding a likelihood of a presence of a region of interest in the captured images; and
a classification means configured to classify, based on the score, the captured images in time series if a predetermined condition is satisfied,
wherein, upon detecting a variation in the captured images, the score calculation means is configured to set a time after the variation as the start time.

[Supplementary Note 2]

The image processing device according to Supplementary Note 1,
wherein the score calculation means is configured to detect the variation, based on a degree of similarity between

the captured image obtained at a current processing time and
the captured image obtained at a processing time immediately preceding the current processing time.

[Supplementary Note 3]

The image processing device according to Supplementary Note 1,
wherein the score calculation means is configured to detect the variation, based on

a confidence level regarding the presence of the region of interest in the captured image obtained at a current processing time and
a confidence level regarding the presence of the region of interest in the captured image obtained at a processing time immediately preceding the current processing time.

[Supplementary Note 4]

The image processing device according to any one of Supplementary Notes 1 to 3,
wherein, even in a case where the variation in the captured images is detected, if the score is close within a predetermined value to a threshold value for executing the classification, the score calculation unit is configured to stop to set the time after the variation as the start time.

[Supplementary Note 5]

The image processing device according to Supplementary Note 4,
wherein, in a case where the variation in the captured images is detected and the score is close within the predetermined value to the threshold value,
the score calculation means is configured to:

determine whether or not the score shifts to approach the threshold value within a predetermined time;
upon determining that the score shifts to approach the threshold value within the predetermined time, stop to set the time after the variation as the start time; and
upon determining that the score does not shift to approach the threshold value within the predetermined time, set the time after the variation as the start time.

[Supplementary Note 6]

The image processing device according to any one of Supplementary Notes 1 to 5,

wherein the score calculation means is configured to calculate the score, based on a likelihood ratio regarding the presence and an absence of the region of interest in the captured images in time series, and
wherein the classification means is configured to perform the classification regarding the presence and the absence of the region of interest in the captured images in time series.

[Supplementary Note 7]

The image processing device according to any one of Supplementary Notes 1 to 6, wherein if the score reaches a predetermined threshold value, the classification means is configured to determine that the predetermined condition is satisfied and perform the classification.

[Supplementary Note 8]

The image processing device according to any one of Supplementary Notes 1 to 7, wherein, if the predetermined condition is satisfied, or, if the variation is detected, the score calculation means is configured to update the start time and sequentially calculate the score based on the updated start time.

[Supplementary Note 9]

The image processing device according to any one of Supplementary Notes 1 to 7, wherein the score calculation means is configured to set the start time at predetermined time intervals and calculate the scores based on the respective set start times in parallel.

[Supplementary Note 10]

The image processing device according to any one of Supplementary Notes 1 to 9,

wherein the region of interest is a lesion part suspected of a lesion, and
wherein the score calculation means is configured to calculate the score regarding the likelihood of the presence of the lesion part.

[Supplementary Note 11]

The image processing device according to any one of Supplementary Notes 1 to 10, further comprising an output control means configured to output, by a display device or an audio output device, information regarding the score and a classification result by the classification means.

[Supplementary Note 12]

An image processing method executed by a computer, the image processing method comprising:

acquiring a captured image obtained by photographing an examination target by a photographing unit provided in an endoscope;
calculating, based on the captured images in time series obtained from a start time, a score regarding a likelihood of a presence of a region of interest in the captured images;
upon detecting a variation in the captured images, setting a time after the variation as the start time; and
classifying, based on the score, the captured images in time series if a predetermined condition is satisfied.

[Supplementary Note 13]

A storage medium storing a program executed by a computer, the program causing the computer to:

acquire a captured image obtained by photographing an examination target by a photographing unit provided in an endoscope;

calculate, based on the captured images in time series obtained from a start time, a score regarding a likelihood of a presence of a region of interest in the captured images;

upon detecting a variation in the captured images, set a time after the variation as the start time; and

classify, based on the score, the captured images in time series if a predetermined condition is satisfied.

While the invention has been particularly shown and described with reference to example embodiments thereof, the invention is not limited to these example embodiments. It will be understood by those of ordinary skilled in the art that various changes in form and details may be made therein without departing from the spirit and scope of the present invention as defined by the claims. In other words, it is needless to say that the present invention includes various modifications that could be made by a person skilled in the art according to the entire disclosure including the scope of the claims, and the technical philosophy. All Patent and Non-Patent Literatures mentioned in this specification are incorporated by reference in its entirety.

DESCRIPTION OF REFERENCE NUMERALS

1, 1X    Image Processing Device
2    Display device
3    Endoscope
4    Server device
11    Processor
12    Memory
13    Interface
14    Input unit
15    Light source unit
16    Audio output unit
100    Endoscopic examination system

**Claims**

1. An image processing device comprising:

   an acquisition means configured to acquire a captured image obtained by photographing an examination target by a photographing unit provided in an endoscope;
   a score calculation means configured to calculate, based on the captured images in time series obtained from a start time, a score regarding a likelihood of a presence of a region of interest in the captured images; and
   a classification means configured to classify, based on the score, the captured images in time series if a predetermined condition is satisfied,
   wherein, upon detecting a variation in the captured images, the score calculation means is configured to set a time after the variation as the start time.

2. The image processing device according to claim 1,
   wherein the score calculation means is configured to detect the variation, based on a degree of similarity between

   the captured image obtained at a current processing time and
   the captured image obtained at a processing time immediately preceding the current processing time.

3. The image processing device according to claim 1,
   wherein the score calculation means is configured to detect the variation, based on

   a confidence level regarding the presence of the region of interest in the captured image obtained at a current processing time and
   a confidence level regarding the presence of the region of interest in the captured image obtained at a processing time immediately preceding the current processing time.

**4.** The image processing device according to any one of claims 1 to 3,
wherein, even in a case where the variation in the captured images is detected, if the score is close within a predetermined value to a threshold value for executing the classification, the score calculation unit is configured to stop to set the time after the variation as the start time.

**5.** The image processing device according to claim 4,
wherein, in a case where the variation in the captured images is detected and the score is close within the predetermined value to the threshold value,
the score calculation means is configured to:

determine whether or not the score shifts to approach the threshold value within a predetermined time;
upon determining that the score shifts to approach the threshold value within the predetermined time, stop to set the time after the variation as the start time; and
upon determining that the score does not shift to approach the threshold value within the predetermined time, set the time after the variation as the start time.

**6.** The image processing device according to any one of claims 1 to 5,

wherein the score calculation means is configured to calculate the score, based on a likelihood ratio regarding the presence and an absence of the region of interest in the captured images in time series, and
wherein the classification means is configured to perform the classification regarding the presence and the absence of the region of interest in the captured images in time series.

**7.** The image processing device according to any one of claims 1 to 6,
wherein if the score reaches a predetermined threshold value, the classification means is configured to determine that the predetermined condition is satisfied and perform the classification.

**8.** The image processing device according to any one of claims 1 to 7,
wherein, if the predetermined condition is satisfied, or, if the variation is detected, the score calculation means is configured to update the start time and sequentially calculate the score based on the updated start time.

**9.** The image processing device according to any one of claims 1 to 7,
wherein the score calculation means is configured to set the start time at predetermined time intervals and calculate the scores based on the respective set start times in parallel.

**10.** The image processing device according to any one of claims 1 to 9,

wherein the region of interest is a lesion part suspected of a lesion, and
wherein the score calculation means is configured to calculate the score regarding the likelihood of the presence of the lesion part.

**11.** The image processing device according to any one of claims 1 to 10, further comprising
an output control means configured to output, by a display device or an audio output device, information regarding the score and a classification result by the classification means.

**12.** An image processing method executed by a computer, the image processing method comprising:

acquiring a captured image obtained by photographing an examination target by a photographing unit provided in an endoscope;
calculating, based on the captured images in time series obtained from a start time, a score regarding a likelihood of a presence of a region of interest in the captured images;
upon detecting a variation in the captured images, setting a time after the variation as the start time; and
classifying, based on the score, the captured images in time series if a predetermined condition is satisfied.

**13.** A storage medium storing a program executed by a computer, the program causing the computer to:

acquire a captured image obtained by photographing an examination target by a photographing unit provided in an endoscope;

calculate, based on the captured images in time series obtained from a start time, a score regarding a likelihood of a presence of a region of interest in the captured images;
upon detecting a variation in the captured images, set a time after the variation as the start time; and
classify, based on the score, the captured images in time series if a predetermined condition is satisfied.

# FIG. 1

100 : ENDOSCOPIC EXAMINATION SYSTEM

FIG. 2

## FIG. 3

FIG. 4

FIG. 5

FIG. 6

FIG. 7

70

UNDER
DETERMINATION

CURRENT SCORE
−0.5

−1 : THRESHOLD FOR
NO LESION DETECTED

1 : THRESHOLD FOR
LESION DETECTED

FIG. 8

FIG. 9

SCORE IS ON THE RISE

PLEASE SLOW DOWN
OPERATION SPEED

CURRENT SCORE　O. 3
(−1 : THRESHOLD
FOR NO LESION DETECTED
1 : THRESHOLD
FOR LESION DETECTED)

# FIG. 10

START

S11

ACQUIRE CAPTURED IMAGE

S12

CALCULATE VARIATION SCORE BETWEEN
CURRENT PROCESSING IMAGE & PAST IMAGE

S13

IS VARIATION SCORE
LESS THAN THRESHOLD ?

No → S17

RESET
CLASSIFICATION
SCORE

Yes

S14

CALCULATE CLASSIFICATION SCORE

S15

DOES CLASSIFICATION SCORE
REACH THRESHOLD ?

No

Yes

S16

DETERMINATION & DISPLAY REGARDING
PRESENCE / ABSENCE OF LESION PART

END

# FIG. 11

START

S21

START THREAD
FOR CALCULATING CLASSIFICATION SCORE
AT PREDETERMINED INTERVALS

S22

ACQUIRE CAPTURED IMAGE

S23

CALCULATE VARIATION SCORE BETWEEN
CURRENT PROCESSING IMAGE & PAST IMAGE

S24    IS VARIATION SCORE
LESS THAN THRESHOLD ?    No    S28    END THREAD &
START NEW THREAD

Yes

S25

CALCULATE CLASSIFICATION SCORE

S26    DOES CLASSIFICATION SCORE
REACH THRESHOLD ?

No

Yes

S27

DETERMINATION & DISPLAY REGARDING
PRESENCE / ABSENCE OF LESION PART &
END THREAD

END

30

# FIG. 12

1X

30X

ACQUISITION MEANS

31X

SCORE CALCULATION MEANS

32X

CLASSIFICATION MEANS

# FIG. 13

START

S31

ACQUIRE CAPTURED IMAGE OF EXAMINATION TARGET

S32

BASED ON CAPTURED IMAGES IN TIME SERIES OBTAINED FROM START TIME, CALCULATE SCORE REGARDING REGION OF INTEREST

S33

VARIATION IN CAPTURED IMAGES DETECTED ? — No

Yes

S34

SET TIME AFTER VARIATION AS START TIME

S35

PREDETERMINED CONDITION SATISFIED ? — No

Yes

S36

CLASSIFY TIME SERIES CAPTURED IMAGES BASED ON SCORE

END

## INTERNATIONAL SEARCH REPORT

| International application No. |
| --- |
| **PCT/JP2022/008253** |

### A.   CLASSIFICATION OF SUBJECT MATTER

*A61B 1/045*(2006.01)i
FI:   A61B1/045 618

According to International Patent Classification (IPC) or to both national classification and IPC

### B.   FIELDS SEARCHED

Minimum documentation searched (classification system followed by classification symbols)

A61B1/00-1/32

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C.   DOCUMENTS CONSIDERED TO BE RELEVANT

| Category* | Citation of document, with indication, where appropriate, of the relevant passages | Relevant to claim No. |
| --- | --- | --- |
| A | WO 2016/110993 A1 (OLYMPUS CORP  ) 14 July 2016 (2016-07-14)<br>entire text, all drawings | 1-13 |
| A | WO 2020/170791 A1 (FUJIFILM CORP) 27 August 2020 (2020-08-27)<br>entire text, all drawings | 1-13 |
| A | WO 2020/039929 A1 (FUJIFILM CORP) 27 February 2020 (2020-02-27)<br>entire text, all drawings | 1-13 |
| A | WO 2020/067105 A1 (FUJIFILM CORP) 02 April 2020 (2020-04-02)<br>entire text, all drawings | 1-13 |
| A | WO 2020/036109 A1 (FUJIFILM CORP) 20 February 2020 (2020-02-20)<br>entire text, all drawings | 1-13 |
| A | WO 2020/194497 A1 (NEC CORP) 01 October 2020 (2020-10-01)<br>entire text, all drawings | 1-13 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **19 April 2022** | **10 May 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2022/008253**

| Patent document cited in search report | | | Publication date (day/month/year) | Patent family member(s) | | | Publication date (day/month/year) |
|---|---|---|---|---|---|---|---|
| WO | 2016/110993 | A1 | 14 July 2016 | US 2017/0296043 A1 entire text, all drawings | | | |
| WO | 2020/170791 | A1 | 27 August 2020 | US 2021/0350534 A1 entire text, all drawings | | | |
| WO | 2020/039929 | A1 | 27 February 2020 | (Family: none) | | | |
| WO | 2020/067105 | A1 | 02 April 2020 | US 2021/0201486 A1 entire text, all drawings CN 112770662 A | | | |
| WO | 2020/036109 | A1 | 20 February 2020 | US 2021/0153720 A1 entire text, all drawings | | | |
| WO | 2020/194497 | A1 | 01 October 2020 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020003607 A
- WO 2020194497 A